# EUROPEAN PATENT APPLICATION

(11) **EP 3 785 696 A1**
(43) Date of publication of application: **03.03.2021**
(21) Application number: 19193987.5
(22) Date of filing: 28.08.2019
(51) Int. Cl.: A61K 8/43, A61Q 19/02, A61P 1/00, A61P 1/16, A61P 5/38, A61P 17/00, A61P 17/02, A61P 25/00, A61K 8/46, A61K 31/155

(54) **COSMETIC USE OF AROMATIC FORMAMIDINE DERIVATIVES**

(71) Applicant: B.R.A.I.N. Biotechnology Research And Information Network AG, 64673 Zwingenberg (DE)
(72) Inventor: BACK, Robin, 64625 Bensheim (DE); KROHN, Michael, 64653 Lorsch (DE); LANGER, Martin, 76227 Karlsruhe (DE)
(74) Representative: Vossius & Partner Patentanwälte Rechtsanwälte mbB

(57) **Abstract**

The present invention relates to the non-therapeutic cosmetic use of compounds of Formula (I) or of cosmetically acceptable salts thereof, 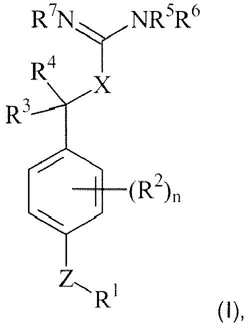 which are useful for skin lightening, brightening of age spots, brightening of freckles, reducing pigmentation of skin and levelling of skin color.

## Description

### Field of the invention

The present invention relates to a compound of Formula (I) including cosmetically acceptable salts thereof, to the non-therapeutic cosmetic use thereof, to cosmetic compositions comprising a compound of Formula (I) including the non-therapeutic cosmetic use thereof. Apart from this non-therapeutic cosmetic aspect, one aspect of the invention also relates to the compound of Formula (I), or a pharmaceutically acceptable salt thereof, for use in the prevention or treatment of a disease or disorder selected from the group consisting of melasma, Addison's disease, Cushing's disease, acanthosis nigricans, Peutz-Jeghers syndrome, smoker's melanosis, Cronkhite-Canada syndrome, Nelson's syndrome, porphyria and haemochromatosis.

It has been found that compounds of Formula (I) have melanogenesis reducing activity. This melanogenesis reducing activity may, e.g., be mediated by antagonizing the melanocortin-1-receptor (MC1-R).

It is thus an object of the present invention to provide improved means and methods for skin lightening (sometimes also referred to as skin whitening), brightening of age spots, brightening of freckles, reducing pigmentation of skin and levelling of skin color.

### Background of the invention

Skin and hair color is determined by the relative amounts of eumelanin, the brown-black pigment, and pheomelanin, the red-yellow pigment, in epidermal and follicular melanocytes, respectively. Pigmentation of mammalian hair and skin depends on various factors and, in particular, environmental factors (seasons of the year), race, sex and age. The melanocytes are highly differentiated dendritic cells which synthesize melanin by means of specific organelles, the melanosomes, which are, based on their final colour, distinguished as pheo- and eumelanosomes, respectively. These melanosomes progress through a series of defined developmental stages (I-IV) within melanocytes, beginning as unpigmented precursors and ending as a membrane-enclosed bag of melanin. The melanin is later distributed to keratinocytes via the melanocyte dendrites.

Within the lumen of melanosomes, eumelanin is synthesized from the intermediate dopaquinone by successive hydroxylation, oxidation and carboxylation reactions. In the case of pheomelanin at least one cysteine dependent reduction step (use of cysteinyldopa) is needed. An overview of the mechanisms of melanogenesis and skin pigmentation is given in e.g. Lin, J. Y. and Fisher, D. E. (2007) Melanocyte biology and skin pigmentation, Nature 445, 843-50; or Brenner, M. and Hearing, V. J. (2008) Modifying skin pigmentation - approaches through intrinsic biochemistry and exogenous agents, Drug Discov Today Dis Mech. 2008;5(2):e189-e199).

Many genes involved in melanogenesis have been identified through comparative genomic studies of mouse coat color mutations and by the molecular characterisation of human hypopigmentary genetic diseases such as albinism of which the major forms are described as oculocutaneous albinism type 1, 2 and 3 (OCA1 - 3).

A key role in regulation of melanogenesis has been attributed to the melanocortin receptor 1 (MC1-R) which is one out of five members (MC1-R - MC5-R) belonging to the family of G-protein coupled melanocortin receptors (Beaumont KA et al. (2007) Receptor function, dominant negative activity and phenotype correlations for MC1-R variant alleles, Hum Mol Genet 16(18), 2249-60).

Agonistic ligands of this receptor family are, e.g., the adrenocorticotropic hormone (ACTH), alpha-melanocortin stimulating hormone (alpha-MSH), beta-MSH, gamma-MSH, corticotropin-like intermediate lobe peptide (CLIP), beta-lipotropin and beta-endorphin. These peptide hormones are all processed posttranslationally by various prohormone convertases from a 31-36 kD precursor protein, the pre-prohormone proopiomelanocortin (POMC).

The melanocortin receptors are involved in a variety of physiological aspects and their function is linked to pigmentation in case of MC1-R, steroidogenesis (MC2-R), energy homeostasis (MC3-R), energy homeostasis and erectile activity (MC4-R) and sebaceous gland lipid secretion (MC5-R). All of these receptors are having alpha-MSH as their preferred ligand with the exception of MC2-R where ACTH is the most potent ligand. For the receptors MC1-R, MC3-R and MC4-R the peptides agouti and agouti related protein (AgRP) are described as antagonistic ligands. Due to the diversity of physiological functions of the melanocortin receptors a vast area of applications is implicated. Thus, novel bioactive compounds which interfere with the endogenous signal transduction of these receptors will have a potential in modulation of skin and hair pigmentation, treatment of obesity, regulation of fat consumption, sexual dysfunction, acne, pain and memory dysfunction, skin cancer and inflammatory diseases (see e.g.: MacNeil DJ, Howard AD, Guan X, Fong TM, Nargund RP, Bednarek MA, Goulet MT, Weinberg DH, Strack AM, Marsh DJ, Chen HY, Shen CP, Chen AS, Rosenblum CI, MacNeil T, Tota M, MacIntyre ED, Van der Ploeg LH. (2002), The role of melanocortins in body weight regulation: opportunities for the treatment of obesity. Eur J Pharmacol. Aug 16; 450(1):93-109; Samama, P., Rumennik, L., Grippo, J.F. (2003) The melanocortin receptor MCR4 controls fat consumption. Regul Pept. 113:85-88).

The melanocyte responds to the peptide hormones alpha-melanocortin stimulating hormone (alpha-MSH) or adrenocorticotropic hormone (ACTH), and, to a lesser extent, to beta-MSH and gamma-MSH through the MC1-R G-protein coupled receptor (melanocortin receptor 1) to stimulate melanin production. It is confirmed that all members of the melanocortin receptor family couple to the adenylyl cyclase/protein kinase A (PKA) intracellular signaling pathway (Scott M. et al. (2002) Human melanocortin 1 receptor variants, receptor function and melanocyte response to UV radiation, Journal of Cell Science 115, 2349-2355; Abdel-Malek ZA et al. (2009) Alpha-MSH tripeptide analogs activate the melanocortin 1 receptor and reduce UV-induced DNA damage in human melanocytes. Pigment Cell Melanoma Res. 22(5):635-644).
The pheomelanosome, containing the tyrosinase, a key enzyme of melanogenesis, produces light red/yellowish melanin, whereas the eumelanosome produces darker melanins via induction of additional TYRP1 (Tyrosinase Related Protein 1), TYRP2 (Tyrosinase Related Protein 2), SILV (silver) enzymes, and the P-protein. Intramelanosomal pH governed by the P-protein may act as a critical determinant of tyrosinase enzyme activity to control the initial step in melanin synthesis or TYRP complex formation to facilitate melanogenesis and melanosomal maturation (see review by: Sturm, R.A., Teasdale, R.D. and Box, N.F. (2001) Human pigmentation genes: identification, structure and consequences of polymorphic variation. Gene, Volume 277, Issues 1-2, 17: 49-62). The expression of P-protein can be used as indicator for inhibition or activation of melanogenesis; e.g. US-patent 7,604,949 and EP-patent 1190248 B1 disclose a screening method for compounds affecting melanogenesis based on the expression of the P-protein encoding gene.

Functional correlation of MC1-R alleles with more than 30 known variants within skin and hair colour provides evidence that this receptor molecule is a key component underlying normal human pigment variation. Among the approximately 70 genes involved in melanogenesis the tyrosinase, the two tyrosinase-related proteins TRP-1 (an oxidase) and TRP-2 (tautomerase) together with the melanocortin receptor 1 are regarded as the major regulators of mammalian melanogenesis, which is up to now only partially understood.

Moreover, it has been shown that the beta isoform of the Protein Kinase C (PKC) modulates human melanogenesis through tyrosinase activation. From homologous phosphorylation domains of the cytoplasmatic tail of the tyrosinase as well as of the TRPs it can be concluded that phosphorylation of the C-terminus is involved in the modulation of tyrosinase activity. It is further known from co-cultivation studies that the interplay of melanocytes and keratinocytes affects the level of e. g. the TRPs in melanocytes. However, the regulation of the keratinocyte-melanocyte interactions and the mechanism of melanosome transfer into keratinocytes are not yet completely understood.

Skin coloring has been of concern to human beings for many years. In particular, the ability to generate a tanned appearance without incurring photodamage due to solar radiation is important to many individuals. There have been several methods proposed to accomplish depigmentation (kojic acid, hydroquinone, retinoids), as well as to "tan" the skin without exposure to the sun (dihydroxyacetone and like chemical compounds). Likewise, the ability to remove hyperpigmentation, such as found in age spots, freckles or aging skin generally, or even general body whitening is of interest to individuals desiring a uniform complexion. There are also pigmentation disorders such as, for example melasma, characterized by dark patches or freckles on the skin, and vitiligo, characterized by patches of skin with lack of pigmentation, which can be made less obvious by lightening the tone of the surrounding normal skin. Furthermore, hyperpigmentation is frequently related to inflammation, skin injuries, sun damage and acne vulgaris that is desirable to treat. Also desirable is the treatment of pigmentation disorders associated with diseases such as, e.g., Addison's disease or Cushing's disease. Despite existing artificial bleaching techniques/products which reduce pigmentation of skin and/or hair locally or extensively, the modulation of pigmentation via the natural route, i.e. the modulation of melanogenesis, is considered the best suited approach.

A number of substances and dermatological formulations have been described to modulate melanogenesis in terms of reducing pigmentation of the skin. E.g. WO 2009/064493 describes tricyclic compounds as melanogenesis modifiers for the treatment of aberrant melanogenesis activity. EP 0 526 502 B1 discloses a pharmaceutical composition for depigmentation which blocks melanin synthesis by inhibiting the metabolic pathway involving tyrosinase. US 5,470,567 discloses a synergistic skin depigmentation composition comprising 4-hydroxyanisole and a retinoid and EP 0 585 130 B1 describes a suppressive effect of tranexamic acid on melanin production. WO 1997/002807 A1 describes a plant extract for skin depigmentation purposes and WO 2008/104532 A1 a topical composition comprising fluocinolone acetonide for depigmentation of the skin.

It is therefore an object of the present invention to provide alternative and/or improved means and methods for skin lightening, brightening of age spots, brightening of freckles, reducing pigmentation of skin and/or levelling of skin color.

The aforementioned object(s) can be achieved by providing the embodiments characterized in the claims, in particular by using a compound of Formula (I).

### Summary of the invention

It is thus an object of the present invention to provide improved means and methods for skin lightening, brightening of age spots, brightening of freckles, reducing pigmentation of skin and/or levelling of skin color.

Accordingly, the present invention relates to the non-therapeutic cosmetic use of a compound of Formula (I), or a cosmetically acceptable salt thereof, 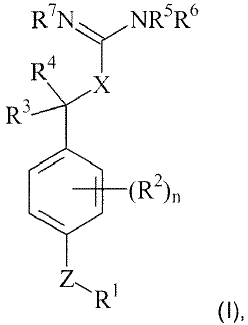 wherein:
X and Z are independently
   - oxygen, or
   - sulfur;
R¹ is
   - a C₁₋₃ alkyl group which is optionally substituted by
      ∘ one or more halogen atoms;
each R² is independently
   - a halogen, or
   - a C₁₋₃ alkyl group which is optionally substituted by
      ∘ one or more halogen atoms;
R³ and R⁴ are each independently
   - a hydrogen atom,
   - a C₁₋₃ alkyl group which is optionally substituted by
      ∘ one or more halogen atoms, or
   - a halogen atom;
R⁵, R⁶ and R⁷ are each independently
   - a hydrogen atom, or
   - a C₁₋₃ alkyl group which is optionally substituted by
      ∘ one or more halogen atoms, or
n is 0, 1, 2 or 3.

Furthermore, the present invention relates to a composition comprising
- 0.01 to 5 wt-% of a compound of Formula (I) as defined above, or a cosmetically acceptable salt thereof; and
- a cosmetically acceptable base.

Still further, the invention relates to the non-therapeutic cosmetic use of a composition as described above.

In a separate embodiment, the present invention also relates to a compound of Formula (I) as defined herein, or a pharmaceutically acceptable salt thereof, for use in the prevention or treatment of a disease or disorder selected from the group consisting of melasma, Addison's disease, Cushing's disease, acanthosis nigricans, Peutz-Jeghers syndrome, smoker's melanosis, Cronkhite-Canada syndrome, Nelson's syndrome, porphyria and haemochromatosis.

Moreover, the present invention relates to a compound of Formula (I) as defined herein, or a cosmetically acceptable salt thereof.

### Description of the figures

- **Figure 1:**: Macroscopic Images of MelanoDerm tissues after 14 days of treatment in triplicates: (A) negative control, (B) positive control, (C) compound of Formula (III).
- **Figure 2:**: Antagonistic activity of compound of Formula (III) on all members of the Melanocortin receptor family, determined via a recombinant cell-based *in vitro* assay.

### Definitions

### Cosmetic composition

The term "cosmetic composition" delimits the composition according to the invention from pharmaceutical compositions. The terms "cosmetic composition" and "pharmaceutical composition" are with regard to their purposes, i.e. cosmetic and curative, mutually exclusive. In other words, a cosmetic composition according to the invention is for use in non-therapeutic applications, e.g., directed to beautifying, promoting attractiveness or altering the appearance. In accordance with the present invention, the term "cosmetic composition" relates to a composition for application and/or administration to a subject, preferably a human subject. The cosmetic composition of the invention may consist of or comprise one or more of the compounds recited herein. It may comprise further constituents. For example, other skin whitening ingredients including botanical extracts that contain components that inhibit melanin production in skin such as licorice extract, pomegranate extract, hinokitiol, protocatechuic acid, NAB asafetida (Ferula Foetida) extract, resveratrol, ferulic acid, viniferol, or botanical extract combinations. The composition may, optionally, comprise further molecules capable of altering the characteristics of the compounds of the invention thereby, for example, stabilizing, modulating and/or activating their function. The composition may be in solid or liquid form and may be, inter alia, in the form of (a) powder(s), (a) tablet(s), (a) solution(s) or (an) aerosol(s), and preferably in the form of topical compositions. The cosmetic composition of the present invention may, optionally and additionally, comprise a carrier which may be a cosmetically acceptable carrier. Examples of suitable cosmetically carriers are well known in the art and include phosphate buffered saline solutions, water, emulsions, such as oil/water emulsions, various types of wetting agents, sterile solutions, organic solvents including DMSO etc. Preferably, in case a topic application is envisaged, the cosmetic composition can comprise, without limitation, one or more further ingredients such as keratolytic compounds (e.g. salicylic acid, urea), moisturizers (e.g. artificial or natural oils), anti-irritants (e.g. allantoin, α-bisabolole), anti-oxidants (e.g. tocopherole), chelating agents (e.g. EDTA), preservative agents (e.g. hydroxybenzoic acid and derivatives thereof), sun protection agents (e.g. benzophenone-3), pigments (e.g. titanium dioxide) and fragrances. Also, the cosmetic composition may contain one or more surfactants, preferably one or more anionic surfactants. Preferred surfactants comprise a hydrophobic moiety, such as a carbon chain having about 8 to about 30 carbon atoms, more preferably about 12 to about 20 carbon atoms, and further a hydrophilic moiety, such as sulfate, sulfonate, carbonate, phosphate or carboxylate. The term "carbon chain" includes alkyl groups as well as alkenyl and alkynyl groups. Alkenyl groups include the hydrophobic moiety comprised in unsaturated fatty acids. Preferred anionic surfactants are alkali salts of fatty acids. The hydrophobic carbon chain may also be etherified, such as with ethylene oxide or propylene oxide, to impart a particular physical property, such as increased water solubility or reduced surface tension to the anionic surfactant. Compositions comprising such carriers can be formulated by well known conventional methods. These cosmetic compositions can be administered or applied to the subject at a suitable dose. Application (also referred to herein interchangeably as administration) of cosmetic compositions may be effected by different ways, e.g., topical, oral, intranasal or intrabronchial administration. Preferably, the administration of cosmetic compositions is effected via non-invasive techniques such as topical application/delivery as described below. It is particularly preferred for cosmetic compositions that said administration is carried out by topical delivery, i.e., to a site of hyperpigmentation of the skin. In this case, administration can be effected by rubbing, pouring, sprinkling or spraying on, to effect the introduction into the skin.

In a cosmetic composition the content of the active ingredient(s) can be in a range of 0.00001 to 50 wt-%, more preferred from 0.001-10 wt-% or even more preferred 0.01 to 5 wt-%.

Where desired, the compounds of the invention may be used in combination with one or more other types of non-therapeutic or therapeutic agents which may be applied/administered of the same type (e.g. topically) in the same dosage form (e.g. topically), in a separate dosage form or by a different application form (e.g. orally).

### Hyperpigmentation

The term "hyperpigmentation" is well-known in the art to relate to the darkening of skin and nails by an increase in melanin production in a subject. It is, however, also envisaged that cosmetic use in accordance with the invention is desired by populations exhibiting a darker complexion due to genetic predisposition such as, e.g. subjects from Asia, Africa, South America or of African or Asian, South American descent.

As evident from the above exemplary mentioned conditions, there are many instances in which a subject may be afflicted with hyperpigmentation necessitating, from a cosmetic standpoint and from the personal standpoint of the afflicted subject, a cosmetic use. Corresponding cosmetic use does not include use of hyperpigmentation when considered to be a symptom of an underlying disease. If hyperpigmentation occurs as a symptom of a disease or resulting from a disease, it is not subject to cosmetic use in accordance with the invention prior to the hyperpigmentation causing disease being cured. Also envisaged is the levelling of the tone of skin patches with different pigmentation by depigmentation of the normal not-affected skin surrounding the light skin areas with reduced melanin production.

The topical application of the compounds or compositions of the present invention preferably brings about depigmentation of the body part to which said cosmetic composition is applied to. The body part may include skin, hair and/or eyes of a human subject or of an animal.

### Detailed description of the invention

In summary, the present invention relates to a compound of Formula (I) including cosmetically acceptable salt thereof, to the non-therapeutic cosmetic use thereof, to cosmetic compositions comprising a compound of Formula (I) including the non-therapeutic cosmetic use thereof. Apart from this non-therapeutic cosmetic aspect, one separate aspect of the invention also relates to the compound of Formula (I), or a pharmaceutically acceptable salt thereof, for use in the prevention or treatment of a disease or disorder selected from the group consisting of melasma, Addison's disease, Cushing's disease, acanthosis nigricans, Peutz-Jeghers syndrome, smoker's melanosis, Cronkhite-Canada syndrome, Nelson's syndrome, porphyria and haemochromatosis.

The melanogenesis reducing activity of the compounds of the present invention can, e.g., be determined by assay systems using human epidermal melanocytes and human keratinocytes.

### The compound of Formula (I)

The structural formula of the compound of Formula (I) of the present invention is as follows: 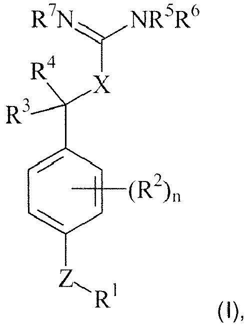
X and Z are independently oxygen or sulfur. Preferably, X is sulfur with Z preferably being oxygen.
R¹ is a C₁₋₃ alkyl group which is optionally substituted by one or more halogen atoms. Preferably, R¹ is a C₁₋₃ alkyl group.
Each R² is independently selected from a halogen and a C₁₋₃ alkyl group which is optionally substituted by one or more halogen atoms. Preferably, R² is halogen. More preferably, R² is bromine.
n is 0, 1, 2 or 3. Preferably, n is 0, 1 or 2. More preferably, n is 0 or 1. Most preferable, n is 1.
It is preferred that there be at least one R² which is in ortho position to the residue "Z-R¹". In a preferred example, there is only one R² which bromine.
R³ and R⁴ are each independently selected from a hydrogen atom, a C₁₋₃ alkyl group which is optionally substituted by one or more halogen atoms, and a halogen atom. Preferably, both R³ and R⁴ are hydrogen.
R⁵, R⁶ and R⁷ are each independently selected from a hydrogen atom, and a C₁₋₃ alkyl group which is optionally substituted by one or more halogen atoms. Preferably, R⁵, R⁶ and R⁷ are each independently a hydrogen atom or a methyl group. Most preferably, R⁵, R⁶ and R⁷ are each hydrogen.

A more preferred example of the compound of Formula (I) is represented by Formula (II) 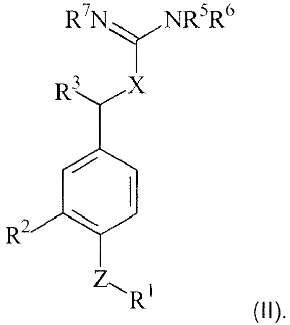
X and Z are independently oxygen or sulfur. Preferably, X is sulfur. Further preferably, Z is oxygen.
R¹ is a C₁₋₃ alkyl group which is optionally substituted by one or more halogen atoms. Preferably, R¹ is a C₁₋₃ alkyl group.
R² is a halogen, or a C₁₋₃ alkyl group which is optionally substituted by one or more halogen atoms. Preferably, R² is a halogen atom.
R³ is a hydrogen atom, or a methyl group. Preferably, R³ is hydrogen.
R⁵, R⁶ and R⁷ are each independently a hydrogen atom or a methyl group. Preferably, R⁵, R⁶ and R⁷ are each hydrogen.

A most preferred example of the compound of Formula (I) is represented by Formula (III)

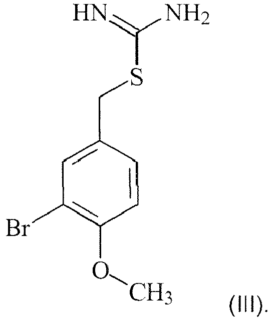

The compound of Formula (III) has the PubChem CID 3094575 and is commercially available, e.g., from Aurora Fine Chemicals LLC as A08.519.431 or ZINC as ZINC4008325.

According to the present invention the compound of Formula (I) and its preferred embodiments include all isomers, stereoisomers and diastereomers. If the compounds are chiral, the present invention is intended to cover racemates, mixtures of the stereoisomers in varying ratios as well as the chiral compounds as such.

The compounds of Formula (I) may, e.g., antagonize the melanocortin-1-receptor (MC1-R). Preferably, they may also selectively antagonize the melanocortin-1-receptor (MC1-R).

### The non-therapeutic cosmetic use of the compounds of Formula (I)

The compounds of Formula (I) are preferably used for one or more selected from the group consisting of:
- skin lightening,
- brightening of age spots,
- brightening of freckles,
- reducing pigmentation of skin and
- levelling of skin color.

These non-therapeutic cosmetic uses of the compounds of Formula (I) do not comprise any uses in or methods for treatment of the human or animal body by surgery or therapy and diagnostic methods practiced on the human or animal body.

The preferred use of the compounds of Formula (I) is in skin lightening. In the present invention, the terms "skin whitening" and "skin lightening" are used interchangeably.

### The composition comprising the compounds of Formula (I) and its non-therapeutic use

The composition according to the present invention comprises 0.01 to 5 wt-% of a compound of Formula (I) or a cosmetically acceptable salt thereof; and a cosmetically acceptable base. This composition preferably further comprises at least one additional cosmetic ingredient.

Furthermore, the invention provides the non-therapeutic cosmetic use of such a composition wherein the use is preferably one or more selected from the group consisting of:
- skin lightening,
- brightening of age spots,
- brightening of freckles,
- reducing pigmentation of skin and
- levelling of skin color.

It is particularly preferred that the compound of Formula (I) and the composition comprising it be applied topically, especially locally.

These non-therapeutic cosmetic uses of a composition comprising the compounds of Formula (I) do not comprise any uses in or methods for the treatment of the human or animal body by surgery or therapy and diagnostic methods practiced on the human or animal body.

The compounds and compositions according to the invention can be applied via all forms normally utilized for the topical route of application including solutions, gels, lotions creams, ointments, foams, mousses, emulsions, microemulsions, milks, serums, aerosols, sprays, dispersions, microcapsules, vesicles and microparticles thereof.

They may also be formulated as solid preparations constituting soaps or cleansing bars. These compositions are formulated according to conventional techniques and typically include a cosmetically acceptable base as described herein.

### The compounds of Formula (I) for use in medicine

In one aspect, the present invention relates to the compound of Formula (I) or a pharmaceutically acceptable salt thereof, for use in the prevention or treatment of a disease or disorder selected from the group consisting of melasma, Addison's disease, Cushing's disease, acanthosis nigricans, Peutz-Jeghers syndrome, smoker's melanosis, Cronkhite-Canada syndrome, Nelson's syndrome, porphyria and haemochromatosis.

Examples of pharmaceutically acceptable salts are well known in the relevant art. The "pharmaceutically acceptable salt" to be used in accordance with the present invention may also be chosen from the same types of salts as the "cosmetically acceptable salt" described herein.

This aspect of the invention is clearly distinct from the cosmetic and non-therapeutic compounds, compositions and uses according to the invention, as can be seen from the definitions of the terms "cosmetic composition" and "pharmaceutical composition" described herein.

### Derivatives

The compositions of the present invention may comprising additionally or alternatively one or more derivatives of the compounds described herein and exhibiting the same or enhanced antagonistic activity as the compound from which they are derived. Exemplary methods to determine whether said antagonistic activity is preserved in a derivative can be determined by the skilled person on the basis of technically straight-forward tests, e.g., using the one disclosed herein or bioassays for the modulation of melanogenesis as disclosed e.g. in EP 1 568 781 B1 using S91 or B16F10 melanoma cells or in the MatTek's MelanoDerm™ System (MatTek Corporation, Ashland, MA 01721).

The compounds recited herein may form salts which are also within the scope of this invention. Reference to a compound of the invention is understood to include reference to salts thereof, unless otherwise indicated. The term "salt(s)", as employed herein, denotes acidic and/or basic salts formed with inorganic and/or organic acids and bases.

The compounds recited herein typically contain a basic moiety may form salts with a variety of organic and inorganic acids. Exemplary acid addition salts include acetates, adipates, alginates, ascorbates, aspartates, benzoates, benzenesulfonates, bisulfates, borates, butyrates, citrates, camphorates, camphorsulfonates, cyclopentanepropionates, digluconates, dodecylsulfates, ethanesulfonates, fumarates, glucoheptanoates, glycerophosphates, hemisulfates, heptanoates, hexanoates, hydrochlorides, hydrobromides, hydroiodides, 2-hydroxyethanesulfonates, lactates, maleates, methanesulfonates, 2-naphtalenesulfonates, nicotinates, nitrates, oxalates, pectinates, persulfates, 3-phenylpropionates, phosphates, picrates, pivalates, propionates, salicylates, succinates, sulfates, sulfonates, tartrates, thiocyanates, toluenesulfonates, such as tosylates, undecanoates, and the like.

To the extent that compounds recited herein and salts thereof may exist in a tautomeric form, all such tautomeric forms are contemplated herein as part of the present invention.

All stereoisomers of the present compounds, such as those which may exist due to asymmetric carbons on the various substituents, including enantiomeric forms (which may exist even in the absence of asymmetric carbons) and diastereomeric forms, are contemplated within the scope of this invention. Individual stereoisomers of the compounds of the invention may, for example, be substantially free of other isomers, or may be admixed, for example, as racemates or with all other, or other specific, stereoisomers.

### The carrier or cosmetically acceptable base

The carrier is preferably a carrier which is suitable for topical application to the keratinous tissue, has good aesthetic properties, is compatible with the active ingredients used in the present invention and any other components, and will not cause safety or toxicity concerns.

The carrier can be in a wide variety of forms. For example, emulsion carriers, including, but not limited to, oil-in-water, water-in-oil, water-in-oil-in-water, and oil-in-water-in-silicon emulsions, are useful herein. As will be understood by the skilled artisan, a given component will distribute primarily into either the water or oil/silicon phase, depending on the water solubility/dispersibility of the component in the composition. A suitable amount of carrier is from about 50% to about 99.99%, more preferably from about 75% to about 99.95%.

The carrier may be in the form of a cosmetically acceptable base. The cosmetically acceptable base is typically in a cream form. Vanishing cream bases typically comprise 1 to 25%, preferably 5 to 25%, more preferably 5 to 20% by weight fatty acids, such as C₁₂₋₂₀ or C₁₄₋₁₈ fatty acids, more preferably one or both of stearic acid and palmitic acid. in addition, the base preferably comprises 0.1 to 12%, more preferably 0.1 to 4% by weight soap which is typically a mixture of alkali metal salts of fatty acids, such as sodium or potassium salts, e.g. of palmitic acid. Furthermore, the cosmetically acceptable base typically comprises water in an amount of 40 to 85% by weight of the cosmetically acceptable base.

Cosmetically acceptable bases are typically comprised in the composition of the invention by 10 to 99.9%, preferably from 50 to 99% by weight of the composition.

Preferred cosmetic compositions have a water content, relative to the total weight of the composition, of 30 to 82 wt. %, preferably 35 to 75 wt. %, in particular 40 to 55 wt. %.

### Additional cosmetic ingredients

The cosmetic compositions of the present invention may include cosmetically acceptable solubilizing or emulsifying agents, binders, buffers, carriers, diluents, disintegrants, emollients, humectants, lipid complexes, liposomes, lubricants, neutral or cationic lipids, penetration enhancers, powders, preservatives, skin protectants, solvents, surface active agents, thickeners, UV absorption agents (sunscreens), and other cosmetically acceptable carriers or excipients. These may also be referred to as additional cosmetic ingredients.

The person skilled in the art is well aware of additional cosmetic ingredients and methods and means to formulate the compounds of the invention into suitable cosmetic compositions according to relevant handbooks on cosmetics or pharmaceutical compositions (e.g. Handbook of cosmetic science and technology, Andre Barel, Marc Paye, Howard I. Maibach (eds.), Marcel Dekker Inc., New York; Grundlagen und Rezepturen der Kosmetika, Schrader, K., Huthig Buch Verlag, Heidelberg, 2nd Edition, 1989; Handbook of Pharmaceutical Manufacturing Formulations, Second Edition, Part II. Manufacturing Formulations, Niazi, S., Informa Healthcare, ISBN: 9781420081060; Formulation Technology: Emulsions, Suspensions, Solid Forms, Hans Mollet, Wiley-VCH; 1st Edition (2001), ISBN-10: 3527302018; Lehrbuch der Pharmazeutischen Technologie: Mit einer Einführung in die Biopharmazie, Kurt H. Bauer, Wissenschaftliche Verlagsgesellschaft; 8th edition, July 2006, ISBN-10: 3804722229, International Cosmetic Ingredient Dictionary and Handbook, Sixteenth Edition 2016, by Personal Care Products Council, Washington).

Examples of skin protectants include vitamin E oil, allantoin, glycerin, zinc oxide, vitamins A, B (e.g. biotin and pantothenic acid), C, E, F, H, and P, and esters thereof.

Examples of emollients include short chain alkyl or aryl esters (C₁₋₆) of long chain straight or branched chain alkyl or alkenyl alcohols or acids (C₈₋₃₂) and their polyethoxylated derivatives; short chain alkyl or aryl esters (C₁₋₆) of C₄₋₁₂ diacids or diols optionally substituted with one or more -OH; alkyl or aryl C₁₋₁₀ esters of glycerol, pentaerythritol, ethylene glycol, propylene glycol, as well as polyethoxylated derivatives of these and polyethylene glycol; C₁₂₋₂₂ alkyl esters or ethers of polypropylene glycol; C₁₂₋₂₂ alkyl esters or ethers of polypropylene glycol/polyethylene glycol copolymer; and polyether polysiloxane copolymers. Additional examples of emollients include cyclic and linear dimethicones, polydialkylsiloxanes, polyaryl/alkylsiloxanes, C₈₋₃₆ alkyl and alkenyl esters of long (C₈₋₃₆) straight or branched chain alkyl or alkenyl alcohols or acids; C₈₋₃₆ alkyl and alkenyl amides of straight or branched chain C₈₋₃₆ alkyl or alkenyl amines or acids; hydrocarbons including straight and branched chain alkanes and alkenes such as squalene, squalane and mineral oil; jojoba oil polysiloxane polyalkylene copolymers, dialkoxy dimethyl polysiloxanes, C₁₋₆ alkyl or aryl esters of C₁₂₋₂₂ diacids or diols optionally substituted in available positions by OH such as diisopropyl dimer dilinoleate; and C₁₂₋₂₂ alkyl and alkenyl alcohols, C₈₋₃₆ alkyl or aryl esters of C₁₂₋₂₂ diacids or diols optionally substituted with one or more -OH, such as diisostearyl dimer dilinoleate; lanolin and lanolin derivatives, and beeswax and its derivatives. Examples of humectant type emollients include glycerol, propylene glycol, dipropylene glycol, polypropylene glycol, polyethylene glycol, sorbitol, pantothenol and gluconic acid salts.

Penetration enhancers include fatty acids, particularly C₁₀₋₂₆ fatty acids, bile salts, chelating agents and surfactants. Fatty acids and their derivatives include, for example, oleic acid, lauric acid, capric acid, caprylic acid, hexanoic acid, myristic acid, palmitic acid, valeric acid, stearic acid, linoleic acid, linolenic acid, arachidonic acid, oleic acid, elaidic acid, erucic acid, nervonic acid, dicaprate, tricaprate, recinleate, 1-monooleoyl-rac-glycerol, dilaurin, arachidonic acid, glyceryl-monocaprate, 1-dodecylazacycloheptan-2-one, acylcarnitines, acylcholines, mono- and di-glycerides and physiologically acceptable salts thereof, such as oleate, laurate, caprate, myristate, palmitate, stearate and linoleate are known, and can be used as penetration enhancers.

Additional cosmetic ingredients, in particular for topical application, are oily materials such as various fats including oils, waxes, hydrocarbons and ester oils, fatty acids, higher alcohols, metallic soaps, hydrophilic or lipophilic gelling agents, hydrophilic or lipophilic active agents, vitamins, hormones, amino acids, surfactants, colorants, dyes, pigments, fragrances, odor absorbers, antiseptics, preservatives, bactericides, humectants, thickeners, solvents, fillers, antioxidants, sequestering agents, or sunscreens. Any ingredients may be used unless they impair the effects of the present invention.

Examples of suitable oils include mineral oils, plant oils such as peanut oil, sesame oil, soybean oil, safflower oil, sunflower oil, animal oils such as lanolin or perhydrosqualene, synthetic oils such as PurCellin Oil, silicone oils such as cyclomethicone. Fatty alcohols, fatty acids such as stearic acid and waxes such as paraffin wax, carnauba wax or beeswax may also be used as fats.

The composition of the present invention may also contain emulsifying agents such as glyceryl stearate, solvents such as lower alcohols including ethanol, isopropanol, and propylene glycol, hydrophilic gelling agents including carboxyvinyl polymers or acrylic copolymers, polyacrylamides, polysaccharides, lipophilic gelling agents or fatty acid metal salts, hydrophilic acting agents such as amino acids, sugars, starch or urea, lipophilic active agents such as retinol or tocopherol.

Additional compounds for skin lightening may be included and, e.g. be selected from niacin, 1,3-diphenyl propane derivatives, 2-(4-acetoxyphenyl)-1,3-dithane, 2-(4-hydroxyphenoxy)propionic acid, 2-(4-hydroxyphenyl)-1,3-dithane, 2,4-resorcinol derivatives, 2,5-dihydroxybenzoic acid and its derivatives, 3,4,5-trihydroxybenzyl derivatives, 3,5-resorcinol derivatives, 4-hydroxy benzoic acid derivatives, 4-hydroxy-5-methyl- 3[2H]-furanone, 4-hydroxyanisole and its derivatives, 5-octanoyl salicylic acid, aloe extract, ammonium lactate, anethole derivatives, apple extract, arbutin, ascorbic acid, ascorbic acid glycoside salts, ascorbic acid glycosides, ascorbic acid salts, azelaic acid, bamboo extract, bearberry extract, bletilla tuber, bupleurum falcatum extract, burnet extract, butyl hydroxy anisole, butyl hydroxy toluene, Chuanxiong, citrate esters, Dang-Gui, deoxyarbutin, dicarboxylic acids, ellagic acid, escinol, estragole derivatives, extracts of Olea europaea , Fadeout (Pentapharm), Fangfeng, fennel extract, ganoderma extract, gaoben, Gatuline Whitening (Gattlefosse), genistic acid and its derivatives, glabridin and its derivatives, gluco pyranosyl-1-ascorbate, gluconic acid, glycolic acid, green tea extract, hydroquinone, hydroxycaprylic acid, hydroxycarboxylic acids like lactic acid and their salts such as sodium lactate, inorganic ascorbic acid esters, inositol ascorbate, kojic acid, lemon extract, linoleic acid, magnesium ascorbyl phosphate, Melawhite (Pentapharm), morus alba extract, mulberry root extract, niacinamide adapalene, nicotinic acid, organic ascorbic acid esters, parsley extract, phellinus linteus extract, pyrogallol derivatives, resorcinol derivatives, rose fruit extract, salicylic acid, salts of 2-(4-hydroxyphenoxy)propionic acid, salts of organic ascorbic acid esters, saturated or unsaturated linear C₉₋₁₈ dicarboxylic acid (salts), Song-Yi extract, tranexamic acid, vitamins such as vitamin B3, vitamin B6, vitamin B12, vitamin C and vitamin A, and mixtures thereof.

Examples of salts of ascorbic acid which may be used in the present invention are sodium hydrogen ascorbate, sodium ascorbate, potassium hydrogen ascorbate, potassium ascorbate, ammonium ascorbate, ammonium hydrogen ascorbate, calcium ascorbate, calcium hydrogen ascorbate, magnesium ascorbate and magnesium hydrogen ascorbate.

Humectants include glycerin, sorbitol, sodium 2-pyrrolidone-5-carboxylate, soluble collagen, dibutyl phthalate, gelatin.

Powders include chalk, fuller's earth, kaolin, starch, gums, colloidal silica sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, and ethylene glycol monostearate.

UV absorption agents that can be used in the compositions of the present invention include inorganic and organic sunblocks. Organic sunblocks include para-aminobenzoic acid (PABA), PABA esters (glyceryl PABA, amyldimethyl PABA and octyldimethyl PABA), butyl PABA, ethyl PABA, ethyl dihydroxypropyl PABA, benzophenones (oxybenzone, sulisobenzone, benzophenone, and benzophenone-1 to 12), N,N,N-trimethyl-4-(2-oxoborn-3-ylidenemethyl)aniline methyl sulfate, 3,3'-(1,4-phenylenedimethylene)-bis(7,7-dimethyl-2-oxobicyclo-[2.2.1]hept-1-ylmethanesulfonic acid) and the salts thereof, α-(2-oxoborn-3-ylidene)-toluene-4-sulfonic acid and the salts thereof, 4-isopropylbenzyl salicylate 2,4,6-trianilino-(p-carbo-2'-ethylhexyl-1'-oxy)-1,3,5-triazine, 3-imidazol-4-ylacrylic acid and the ethyl esters thereof, polymers of N-{(2 and 4)-[2-oxoborn-3-ylidenemethyl]benzyl}-acrylamide, 2,2'-dihydroxy-4,4'-dimethoxybenzo-phenone-5-sodium sulfonate, 2-cyano-3,3-diphenylacrylic acid-2'-ethylhexyl ester, cinnamates (octyl methoxycinnamate, isoamyl p-methoxycinnamate, octylmethoxy cinnamate, cinoxate, diisopropyl methyl cinnamate, DEA-methoxycinnamate, ethyl diisopropylcinnamate, glyceryl octanoate dimethoxycinnamate and ethyl methoxycinnamate), cinnamate esters, salicylates (homomethyl salicylate, benzyl salicylate, glycol salicylate, isopropylbenzyl salicylate, etc.), anthranilates, ethyl urocanate, homosalate, octisalate, dibenzoylmethane derivatives (e.g., avobenzone), octocrylene, octyl triazone, digalloy trioleate, glyceryl aminobenzoate, lawsone with dihydroxyacetone, ethylhexyl triazone, dioctyl butamido triazone, benzylidene malonate polysiloxane, terephthalylidene dicamphor sulfonic acid, disodium phenyl dibenzimidazole tetrasulfonate, diethylamino hydroxybenzoyl hexyl benzoate, bis diethylamino hydroxybenzoyl benzoate, bis benzoxazoylphenyl ethylhexylimino triazine, drometrizole trisiloxane, methylene bis-benzotriazolyl tetramethylbutylphenol, and bis-ethylhexyloxyphenol methoxyphenyltriazine, 4-methylbenzylidenecamphor, and isopentyl 4-methoxycinnamate.

Many UV absorption agents are commercially available, such as 3,3,5-trimethylcyclohexyl salicylate (Homosalate), 2-hydroxy-4-methoxybenzophenone (Benzophenone-3; Uvinul®M 40, Uvasorb®MET, Neo Heliopan®BB, Eusolex®4360), 2-phenylbenzimidazole-5-sulfonic acid and the potassium, sodium and triethanolamine salts thereof (Phenylbenzimidazole Sulfonic Acid; Parsol®HS; Neo Heliopan® Hydro), 1-(4-tert.-butylphenyl)-3-(4-methoxyphenyl)-propane-1,3-dione (Butyl Methoxydibenzoylmethane; Parsol®1789, Eusolex®9020), ethoxylated 4-aminobenzoic acid ethyl ester (PEG-25 PABA; Uvinul®P 25), 4-dimethylaminobenzoic acid 2-ethylhexyl ester (Octyl Dimethyl PABA; Uvasorb®DMO, Escalol®507, Eusolex®6007), salicylic acid 2-ethylhexyl ester (Octyl Salicylate; Escalol®587, Neo Heliopan®OS, Uvinul®O18), 4-methoxycinnamic acid isopentyl ester (Isoamyl p-Methoxycinnamate; Neo Heliopan®E 1000), 4-methoxycinnamic acid 2-ethylhexyl ester (Octyl Methoxycinnamate; Parsol®MCX, Escalol®557, Neo Heliopan®AV), 2-hydroxy-4-methoxybenzophenone-5-sulfonic acid and the sodium salt thereof (Benzophenone-4; Uvinul®MS 40; Uvasorb®S 5), 3-(4'-methylbenzylidene)-D,L-camphor (4-Methylbenzylidene Camphor; Parsol®5000, Eusolex®6300), 3-benzylidenecamphor (3-Benzylidene Camphor), 2,4-dihydroxybenzophenone (Benzophenone-1; Uvasorb®20 H, Uvinul®400), 1,1'-diphenylacrylonitrile acid 2-ethylhexyl ester (Octocrylene; Eusolex®OCR, Neo Heliopan®Type 303, Uvinul®N 539 SG), o-aminobenzoic acid menthyl ester (Menthyl Anthranilate; Neo Heliopan®MA), 2,2',4,4'-tetrahydroxybenzophenone (Benzophenone-2; Uvinul®D-50), 2,2'-dihydroxy-4,4'-dimethoxybenzophenone (Benzophenone-6), and phenylene-1,4-bis-(2-benzimidazole)-3,3'-5,5'-tetrasulfonic acid-bis-sodium salt (Disodium Phenyl Dibenzimidazole Tetrasulfonate, Neo Heliopan® AP).

Inorganic sunblocks include metal oxides, such as zinc oxide, silica, titanium oxide, or combinations thereof.

UV filters and sunblocks which are allowed according to EU regulations include the following:

| Compound name | CAS No. |
|---|---|
| N,N,N-Trimethyl-4-(2-oxoborn-3-ylidenemethyl) anilinium methyl sulphate | 52793-97-2 |
| Benzoic acid, 2-hydroxy-,3,3,5-trimethylcyclohexyl ester / Homosalate | 118-56-9 |
| 2-Hydroxy-4-methoxybenzophenone / Oxybenzone | 131-57-7 |
| 2-Phenylbenzimidazole-5-sulphonic acid and its potassium, sodium and triethanolamine salts / Ensulizole | 27503-81-7 |
| 3,3'-(1,4-Phenylenedimethylene) bis (7,7-dimethyl-2-oxobicyclo-[2.2.1]hept-1-ylmethanesulfonic acid) and its salts / Ecamsule | 92761-26-7 / 90457-82-2 |
| 1-(4-tert-Butylphenyl)-3-(4-methoxyphenyl) propane-1,3-dione / Avobenzone | 70356-09-1 |
| alpha-(2-Oxoborn-3-ylidene)toluene-4-sulphonic acid and its salts | 56039-58-8 |
| 2-Cyano-3,3-diphenyl acrylic acid, 2-ethylhexyl ester / Octocrilene | 6197-30-4 |
| Polymer of N-{(2 and 4)-[(2-oxoborn-3-ylidene)methyl]benzyl}acrylamide | 113783-61-2 |
| 2-Ethylhexyl 4-methoxycinnamate / Octinoxate | 5466-77-3 |
| Ethoxylated Ethyl-4-Aminobenzoate | 116242-27-4 |
| Isopentyl-4-methoxycinnamate / Amiloxate | 71617-10-2 |
| 2,4,6-Trianilino-(p-carbo-2'-ethylhexyl-1'-oxy)-1,3,5-triazine | 88122-99-0 |
| Phenol, 2-(2H-Benzotriazol-2-yl)-4-Methyl-6-(2-Methyl-3-(1,3,3,3-Tetramethyl-1-(Trimethylsilyl)Oxy)-Disiloxanyl)Propyl | 155633-54-8 |
| Benzoic acid, 4,4-{[6-[[[(1,1-dimethylethyl)amino]carbonyl]phenyl]amino]-1,3-5-triazine-2,4-diyl]diimino}bis-, bis(2-ethylhexyl)ester / Iscotrizinol | 154702-15-5 |
| 3-(4'-Methylbenzylidene)-dl-camphor / Enzacamene | 36861-47-9 / 38102-62-4 |
| 2-Ethylhexyl salicylate / Octisalate) | 118-60-5 |
| 2-Ethylhexyl 4-(dimethylamino)benzoate / Padimate O (USAN:BAN) | 21245-02-3 |
| 2-Hydroxy-4-methoxybenzophenone-5-sulfonic acid (Benzophenone-5) and its sodium salt / Sulisobenzone | 4065-45-6 / 6628-37-1 |
| 2,2'-Methylene bis(6-(2H-benzotriazol-2-yl)-4-(1,1,3,3-tetramethylbutyl)phenol) / Bisoctrizole | 103597-45-1 |
| Sodium salt of 2,2'-bis(1,4-phenylene)-1H-benzimidazole-4,6-disulfonic acid / Bisdisulizole disodium (USAN) | 180898-37-7 |
| 2,2'-(6-(4-Methoxyphenyl)-1,3,5-triazine-2,4-diyl)bis(5-((2-ethylhexyl)oxy)phenol) / Bemotrizinol | 187393-00-6 |
| Dimethicodiethylbenzalmalonate | 207574-74-1 |
| Titanium dioxide (nano) | 13463-67-7[1]/ 1317-70-0[2]/ 1317-80-2[3] |
| Titanium dioxide | 13463-67-7[1]/ 1317-70-0[2]/ 1317-80-2[3] |
| Benzoic acid, 2-[4-(diethylamino)-2-hydroxybenzoyl]-, hexylester | 302776-68-7 |
| 1,3,5-Triazine, 2,4,6-tris(1,1'-biphenyl)-4-yl-, including as nanomaterial | 31274-51-8 |
| Zinc oxide | 1314-13-2 |

The compositions according to the invention may furthermore comprise, relative to the total weight of the composition, 1 to 15 wt. %, preferably 2 to 12 wt. % and in particular 3 to 10 wt. % of at least one alcohol. Preferred alcohols are glycol, 1,2-propylene glycol, 1,3-propylene glycol, 2-methyl-1,3-propanediol, glycerol, 1,2-butylene glycol, 1,3-butylene glycol, 1,4-butylene glycol, pentylene glycols, 1,2-hexanediol, 1,6-hexanediol, 1,2,6-hexanetriol, 1,2-octanediol, 1,8-octanediol, dipropylene glycol, tripropylene glycol, diglycerol, triglycerol, erythritol, sorbitol, polyethylene glycol and mixtures of the above-stated substances.

It is preferred that the composition of the present invention preferably contains at least one monoglycerol fatty acid ester, wherein the fatty acid is preferably a C₆₋₂₄ fatty acid, more preferably a C₁₂₋₁₈ fatty acid.

Furthermore, it is preferred that the composition of the present invention comprises polyglycerol difatty acid esters. Examples thereof include polyglyceryl-2 dipalmitate, polyglyceryl-2 dipalmitoleate, polyglyceryl-2 dioleate, polyglyceryl-2 dicerotinate, polyglyceryl-2 dimelissinate, polyglyceryl-3 dipalmitate, polyglyceryl-3 dipalmitoleate, polyglyceryl-3 dioleate, polyglyceryl-3 dicerotinate, polyglyceryl-3 dimelissinate, polyglyceryl-4 dipalmitate, polyglyceryl-4 dipalmitoleate, polyglyceryl-4 dioleate, polyglyceryl-4 dicerotinate and polyglyceryl-4 dimelissinate. Particularly preferred examples are polyglyceryl-3 dipalmitate, polyglyceryl-3 dicerotinate and polyglyceryl-3 dimelissinate.

It is to be understood that the present invention specifically relates to each and every combination of features and examples described herein, including any combination of general and/or preferred features/examples. In particular, the invention specifically relates to each combination of meanings (including general and/or preferred meanings) for the various groups and variables comprised in formula (I).

In this specification, a number of documents including patent applications and scientific literature are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

The invention will now be described by reference to the following examples which are merely illustrative and are not to be construed as a limitation of the scope of the present invention.

### EXAMPLES

The compounds described in this section are defined by their chemical formulae and their corresponding chemical names. In case of conflict between any chemical formula and the corresponding chemical name indicated herein, the present invention relates to both the compound defined by the chemical formula and the compound defined by the chemical name.

### Example 1

### MelanoDerm™ assay

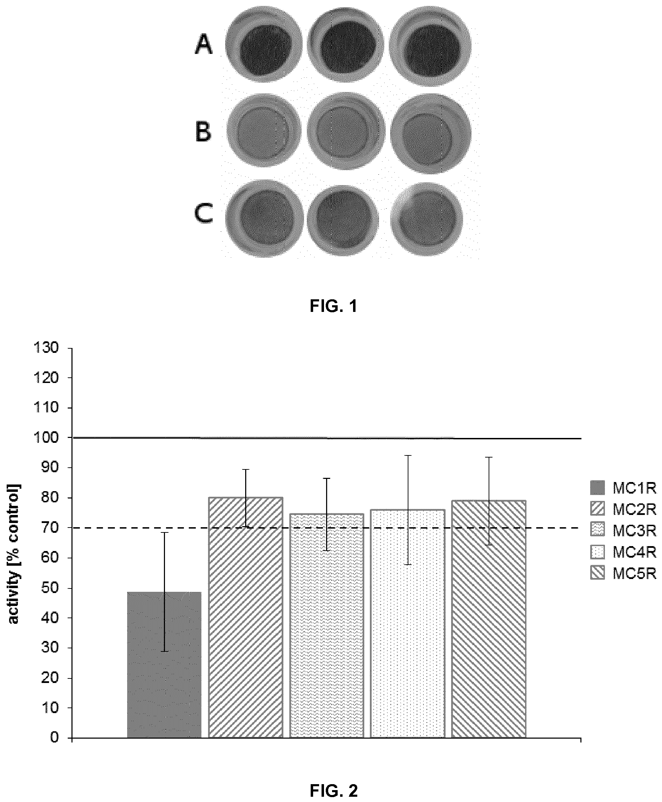

Phenotypic skin lightening was assessed in reconstructed skin using MelanoDerm™ (Mattek Corp.) tissues (Figure 1).

Controls were: negative control (ultrapure water) and positive control (10 µM Ketoconazol). Test compounds were prepared in DMSO with a concentration of 10 mM and diluted in EPI-100-LLM medium (Mattek Corp.) to a final concentration of 25 µM.

MelanoDerm™ tissues were equilibrated in pre-warmed EPI-100-LLMM medium (6-Well plates, 5 ml per well) for 1 h (37°C, 5 % CO₂) before treatment. Test compounds were applied systemically by addition into the culture medium. Dosing and re-feeding of tissues with controls and test compounds continued for a total of 14 days (medium exchange against 5 ml of fresh medium every other day, including addition of respective test compound or control). At the end of the treatment, tissues were analyzed via macroscopic/microscopic images and in vitro assay, for viability and for melanin content. Macroscopic tissue images were examined with respect to skin lightening ability of test compounds compared to negative and positive controls. Cytotoxicity was assessed using microscopic tissue images (10× magnification). Tissue viability as a result of test compound relative to negative control was determined using Cell Proliferation Reagent WST-1 (cat# 05015944001, Roche). For melanin quantitation tissues were submerged for 5 min in phosphate buffered saline (PBS, 5 ml), removed from PBS and rinsed with additional 2 ml PBS. Subsequently, tissues were incubated with 1 % sodium bicarbonate solution (300 µl) for 30 min, then rinsed with PBS (2 x 2 ml), removed from the membrane and dried by blotting on paper. Tissues were suspended in SolvableTM reagent (500ul, cat# 6NE9100, PerkinElmer Inc.) and incubated over night at 60°C. Melanin standard samples with 0, 2.5, 5, 10, 25, 50 and 100 µg/ml (melanin (cat# M8631, Sigma-Aldrich) in SolvableTM) were treated in the same manner. All incubated samples were centrifuged at 13,000 rpm for 5 min and the optical density of the supernatant (200 µl) was measured at 490 nm. The amount of melanin (µg) from each tissue sample was determined using the melanin standard samples calibration curve. Protein concentration of tissue samples was determined using the BCA protein assay kit (Pierce). Finally, the melanin content was normalized on the protein content and then expressed in percent inhibition relative to untreated tissues (negative control, Table 1).

**Table 1: MelanoDerm™ data**

| Percentage of melanin inhibition for compound of Formula (III) in MelanoDerm™ assay | | |
|---|---|---|
| Test Sample | Concentration | % inhibition relative to negative control |
| Positive control: Ketoconazol | 10 µM | -70,9 |
| Compound (III) | 25 µM | -53,4 |

The following Examples 2 to 7 were conducted in order to assess the selectivity of the claimed compounds, more specifically of compound (III). The corresponding data for the testing results of this compound are shown in Fig. 2.

### Example 2: General Methods

### Transfection of HEK293 cells

A MC1-R expressing cell line for assessing melanogenesis modulating activity was established based on engineered HEK 293 cells, stably expressing the murine G-protein Gα15 (NM_010304.3). The promiscuous Gα15 protein couples to MC1-R and therefore links receptor stimulation to Ca²⁺-signaling.

To achieve a stable expression of the target receptor this HEK293/Gα15 cell line was further stably transfected with an expression vector for the human MC1-R receptor. MC1-R cDNA expression results in the corresponding protein (NP_002377.4 with an amino acid replacement at position 105 (A105V)).

### Example 3:

### Determination of compound activity on MC1-R

HEK293 cells stably expressing the target receptor MC1-R and the promiscuous murine Gα15 protein (HEK293/Gα15/MC1-R) were established as described above. MC1-R stimulation leads to increased, MC1-R inhibition to decreased intracellular Ca²⁺-levels. Changes in internal Ca²⁺-concentration are measured via the Ca²⁺-sensitive fluorescent dye Fluo-4 (2-{[2-(2-{5-[bis(carboxymethyl)amino]-2-methyl-phenoxy}ethoxy)-4-(2,7-difluoro-6-hydroxy-3-oxo-3H-xanthen-9-yl)phenyl](carboxymethyl)amino}acetic acid) in a fluorescence plate reader (FLEX-Station, Molecular Devices) at an excitation wavelength of 488nm and emission of 535nm.

Recombinant HEK293/Gα15/MC1-R cells were cultured in DMEM with 10% FBS, 2mM glutamine, 1,3 mg/ml geneticin and 5 µg/ml blasticidin. Cells were seeded in 96 well, poly-D-lysine coated, black well/ clear bottom fluorescence plates in medium without antibiotics. After 24h of growth (37°C, 5 % CO_{2,} saturated humidified atmosphere), cells were stained in Krebs-HEPES buffer (118mM NaCl, 4,7mM KCI, 1,2mM MgSO₄, 1,2mM KH₂PO₄, 4,2mM NaHCO₃, 1,3mM CaCl₂, 10mM HEPES)/ 10mM glucose/ 250µM sulfinpyrazone/ 2µM Fluo-4 AM for 1 h (37°C, 5% CO₂, saturated humidified atmosphere). Subsequently, the buffer is changed against Krebs-HEPES buffer/ 10mM glucose/ 250µM sulfinpyrazone without Fluo-4 and measurement is performed at 37°C. Raw data were analysed using Softmax Pro software (Molecular Devices; algorithm Fₘₐₓ-Fₘᵢₙ/Fₘᵢₙ) and normalized on the αMSH (alpha Melanocyte Stimulating Hormone, positive control) signal.

Test compound was added to cells with a final concentration of 25 µM 10 min prior stimulation with αMSH (using EC80). The threshold for antagonistic activity was defined as 70% of the positive control αMSH (the EC80 value is set as 100%), and the assay was accepted as valid if the standard deviation of the αMSH signal is 10% or less.

For this experiment, it is preferred that a hit reduces the αMSH induced fluorescence signal to 70% or less. It is more preferred if a hit compound reduces the αMSH induced fluorescence signal to 50% or less of the αMSH signal.

For instance, compound (III) was able to reduce MC1R activation by 51,3 %.

### Determination of the MC1-R-selectivity within the melanocortin receptor family

To determine if a compound was selective for MC1-R within the family of melanocortin receptors (five members), it was tested for activity on MC2-R, MC3-R, MC4-R and MC5-R. Since all melanocortin receptors are able to couple to the same G-protein, i.e. murine Gα15, the intracellular signal transduction is comparable for the individual receptor assays.

### Example 4:

### Determination of compound activity on MC2-R:

HEK293 cells stably expressing the target receptor MC2-R, the accessory protein MRAP and the promiscuous murine Gα15 protein were established on basis of the above-mentioned HEK293/Gα15 cell line. These cells were further transfected with an expression vector comprising human MC2-receptor and additional MRAP (MC2-Receptor Accessory Protein) cDNA. MC2-R cDNA expression results in MC2-R protein NP_000520.1, MRAP cDNA expression results in MRAP protein NP_848932.1.

Stimulation of MC2-R with ACTH (adrenocorticotropic hormone) increases intracellular Ca²⁺-concentration, inhibition of MC2-R decreases these ACTH induced Ca²⁺-responses. Changes in intracellular Ca²⁺-levels were measured via the Ca²⁺-sensitive fluorescent dye Fluo-4 in a fluorescent plate reader.

For antagonist-testing, test compound was added to cells with a final concentration of 25 µM 10 min prior addition of ACTH (EC80). The threshold for antagonistic activity was defined in this experiment as 70% of the positive control ACTH (the EC80 value is set as 100%), and the assay was accepted as valid if the standard deviation of the ACTH signal is 10% or less.

### Example 5:

### Determination of compound activity on MC3-R:

HEK293 cells stably expressing the target receptor MC3-R and the promiscuous murine Gα15 protein were established on basis of the above-mentioned HEK293/Gα15 cell line. These cells were further transfected with an expression vector comprising the human MC3-receptor cDNA. MC3-R cDNA expression results in MC3-R protein NP_063941.3.

Stimulation of MC3-R with αMSH increases intracellular Ca²⁺-concentration, inhibition of MC3-R decreases these αMSH induced Ca²⁺-responses. Changes in intracellular Ca²⁺-levels were measured via the Ca²⁺-sensitive fluorescent dye Fluo-4 in a fluorescent plate reader.

For antagonist-testing, test compound was added to cells with a final concentration of 25 µM 10 min prior stimulation with αMSH (using EC80). The threshold for antagonistic activity was defined as 70% of the positive control αMSH (the EC80 value is set as 100%), and the assay was accepted as valid if the standard deviation of the αMSH signal is 10% or less.

### Example 6:

### Determination of compound activity on MC4-R

HEK293 cells stably expressing the target receptor MC4-R and the promiscuous murine Gα15 protein were established on basis of the above-mentioned HEK293/Gα15 cell line. These cells were further transfected with an expression vector comprising the human MC4-receptor cDNA. MC4-R cDNA expression results in MC4-R protein NP_005903.2.

Stimulation of MC4-R with αMSH increases intracellular Ca²⁺-concentration, inhibition of MC4-R decreases these αMSH induced Ca²⁺-responses. Changes in intracellular Ca²⁺-levels were measured via the Ca²⁺-sensitive fluorescent dye Fluo-4 in a fluorescent plate reader.

For antagonist-testing, test compound was added to cells with a final concentration of 25 µM 10 min prior stimulation with αMSH (using EC80). The threshold for antagonistic activity was defined as 70% of the positive control αMSH (the EC80 value is set as 100%), and the assay was accepted as valid if the standard deviation of the αMSH signal is 10% or less.

### Example 7:

### Determination of compound activity on MC5-R

HEK293 cells stably expressing the target receptor MC5-R and the promiscuous murine Gα15 protein were established on the basis of the above-mentioned HEK293/Gα15 cell line. These cells were further transfected with an expression vector comprising the human MC5-receptor cDNA. MC5-R cDNA expression results in MC5-R protein NP_005904.1.

Stimulation of MC5-R with αMSH increases intracellular Ca²⁺-concentration, inhibition of MC5-R decreases these αMSH induced Ca²⁺-responses. Changes in intracellular Ca²⁺-levels were measured via the Ca²⁺-sensitive fluorescent dye Fluo-4 in a fluorescent plate reader.

For antagonist-testing, test compound was added to cells with a final concentration of 25 µM 10 min prior stimulation with αMSH (using EC80). The threshold for antagonistic activity was defined as 70% of the positive control αMSH (the EC80 value is set as 100%), and the assay was accepted as valid if the standard deviation of the αMSH signal is 10% or less.

As shown in Figure 2, compound (III) was able to reduce MC1R activation to about 50% whereas activity of MC2R, MC3R, MC4R, and MC5R was not significantly affected. Thus, compound (III) can be used as selective antagonist of MC1R useful for various purposes such as skin lightening, brightening of age spots, brightening of freckles, reducing pigmentation of skin and levelling of skin color.

## Claims

1. Non-therapeutic cosmetic use of a compound of Formula (I), or a cosmetically acceptable salt thereof, 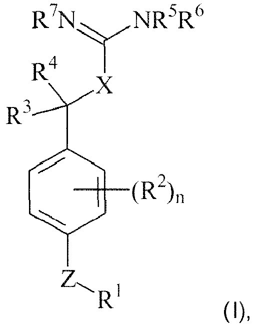 wherein:
X and Z are independently
• oxygen, or
• sulfur;
R¹ is
• a C₁₋₃ alkyl group which is optionally substituted by
∘ one or more halogen atoms;
each R² is independently
• a halogen, or
• a C₁₋₃ alkyl group which is optionally substituted by
∘ one or more halogen atoms;
R³ and R⁴ are each independently
• a hydrogen atom,
• a C₁₋₃ alkyl group which is optionally substituted by
∘ one or more halogen atoms, or
• a halogen atom;
R⁵, R⁶ and R⁷ are each independently
• a hydrogen atom, or
• a C₁₋₃ alkyl group which is optionally substituted by
∘ one or more halogen atoms;
n is 0, 1, 2 or 3.

2. The non-therapeutic cosmetic use according to claim 1, wherein in the compound R⁴ is hydrogen or methyl group.

3. The non-therapeutic cosmetic use according to claim 1 or 2, wherein in the compound n is 1 or 2.

4. The non-therapeutic cosmetic use according to any one of claims 1 to 3, wherein the compound is represented by Formula (II) 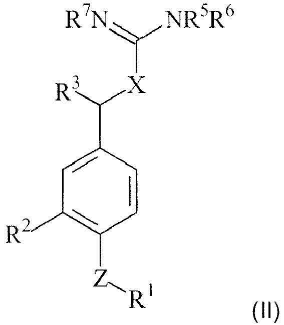 wherein
X and Z are independently
• oxygen, or
• sulfur;
R¹ is
• a C₁₋₃ alkyl group which is optionally substituted by
∘ one or more halogen atoms;
R² is
• a halogen, or
• a C₁₋₃ alkyl group which is optionally substituted by
∘ one or more halogen atoms;
R³ is
• a hydrogen atom, or
• a methyl group;
R⁵, R⁶ and R⁷ are each independently
• a hydrogen atom, or
• a methyl group.

5. The non-therapeutic cosmetic use according to any one of claims 1 to 4, wherein in the compound R¹ is a C₁₋₃ alkyl group.

6. The non-therapeutic cosmetic use according to any one of claims 1 to 5, wherein in the compound R² is a halogen atom.

7. The non-therapeutic cosmetic use according to any one of claims 1 to 6, wherein in the compound R³ is hydrogen.

8. The non-therapeutic cosmetic use according to any one of claims 1 to 7, wherein in the compound R⁵, R⁶ and R⁷ are each independently hydrogen or methyl group.

9. The non-therapeutic cosmetic use according to any one of claims 1 to 8, wherein in the compound X is sulfur, or
wherein in the compound Z is oxygen;
preferably wherein in the compound X is sulfur and Z is oxygen.

10. The non-therapeutic cosmetic use according to claim 1, wherein the compound is represented by Formula (III)

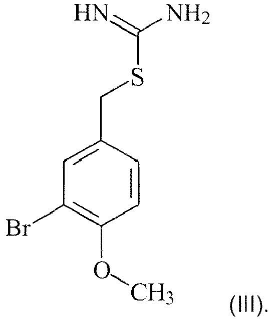

11. The non-therapeutic cosmetic use according to any one of claims 1 to 10, wherein the use is one or more selected from the group consisting of:
• skin lightening,
• brightening of age spots,
• brightening of freckles,
• reducing pigmentation of skin and
• levelling of skin color;
preferably wherein the use is skin lightening.

12. A composition comprising
• 0.01 to 5 wt-% of a compound as defined in any one of claims 1 to 10, or a cosmetically acceptable salt thereof; and
• a cosmetically acceptable base;
preferably wherein the composition further comprises at least one additional cosmetic ingredient.

13. Non-therapeutic cosmetic use of a composition according to claim 12.

14. The non-therapeutic cosmetic use according to claim 13, wherein the use is one or more selected from the group consisting of:
• skin lightening,
• brightening of age spots,
• brightening of freckles,
• reducing pigmentation of skin and
• levelling of skin color;
preferably wherein the use is skin lightening.

15. A compound as defined in any one of claims 1 to 10, or a pharmaceutically acceptable salt thereof, for use in the prevention or treatment of a disease or disorder selected from the group consisting of melasma, Addison's disease, Cushing's disease, acanthosis nigricans, Peutz-Jeghers syndrome, smoker's melanosis, Cronkhite-Canada syndrome, Nelson's syndrome, porphyria and haemochromatosis.

## Amended claims

### Amended claims in accordance with Rule 137(2) EPC.

1. Non-therapeutic cosmetic use a compound of Formula (I), or a cosmetically acceptable salt thereof, 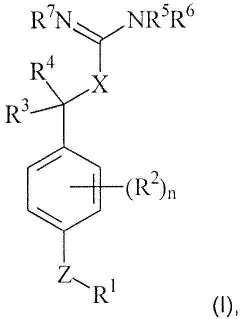 wherein:
X and Z are independently
• oxygen, or
• sulfur;
R¹ is
• a C₁₋₃ alkyl group which is optionally substituted by
∘ one or more halogen atoms;
each R² is independently
• a halogen, or
• a C₁₋₃ alkyl group which is optionally substituted by
∘ one or more halogen atoms;
R³ and R⁴ are each independently
• a hydrogen atom,
• a C₁₋₃ alkyl group which is optionally substituted by
∘ one or more halogen atoms, or
• a halogen atom;
R⁵, R⁶ and R⁷ are each independently
• a hydrogen atom, or
• a C₁₋₃ alkyl group which is optionally substituted by
∘ one or more halogen atoms;
n is 0, 1, 2 or 3,
wherein the use is one or more selected from the group consisting of:
• skin lightening,
• brightening of age spots,
• brightening of freckles,
• reducing pigmentation of skin and
• levelling of skin color;
preferably wherein the use is skin lightening.

2. The non-therapeutic cosmetic use according to claim 1, wherein in the compound R⁴ is hydrogen or methyl group.

3. The non-therapeutic cosmetic use according to claim 1 or 2, wherein in the compound n is 1 or 2.

4. The non-therapeutic cosmetic use according to any one of claims 1 to 3, wherein the compound is represented by Formula (II) 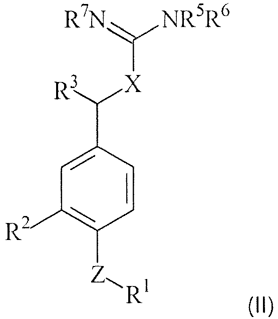 wherein
X and Z are independently
• oxygen, or
• sulfur;
R¹ is
• a C₁₋₃ alkyl group which is optionally substituted by
∘ one or more halogen atoms;
R² is
• a halogen, or
• a C₁₋₃ alkyl group which is optionally substituted by
∘ one or more halogen atoms;
R³ is
• a hydrogen atom, or
• a methyl group;
R⁵, R⁶ and R⁷ are each independently
• a hydrogen atom, or
• a methyl group.

5. The non-therapeutic cosmetic use according to any one of claims 1 to 4, wherein in the compound R¹ is a C₁₋₃ alkyl group.

6. The non-therapeutic cosmetic use according to any one of claims 1 to 5, wherein in the compound R² is a halogen atom.

7. The non-therapeutic cosmetic use according to any one of claims 1 to 6, wherein in the compound R³ is hydrogen.

8. The non-therapeutic cosmetic use according to any one of claims 1 to 7, wherein in the compound R⁵, R⁶ and R⁷ are each independently hydrogen or methyl group.

9. The non-therapeutic cosmetic use according to any one of claims 1 to 8, wherein in the compound X is sulfur, or
wherein in the compound Z is oxygen;
preferably wherein in the compound X is sulfur and Z is oxygen.

10. The non-therapeutic cosmetic use according to claim 1, wherein the compound is represented by Formula (III)

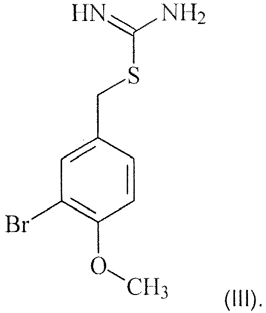

11. A composition comprising
• 0.01 to 5 wt-% of a compound as defined in any one of claims 1 to 10, or a cosmetically acceptable salt thereof; and
• a cosmetically acceptable base;
preferably wherein the composition further comprises at least one additional cosmetic ingredient.

12. Non-therapeutic use of a composition according to claim 11 as a cosmetic.

13. The non-therapeutic cosmetic use according to claim 12, wherein the use is one or more selected from the group consisting of:
• skin lightening,
• brightening of age spots,
• brightening of freckles,
• reducing pigmentation of skin and
• levelling of skin color;
preferably wherein the use is skin lightening.

14. A compound as defined in any one of claims 1 to 10, or a pharmaceutically acceptable salt thereof, for use in the prevention or treatment of a disease or disorder selected from the group consisting of melasma, Addison's disease, Cushing's disease, acanthosis nigricans, Peutz-Jeghers syndrome, smoker's melanosis, Cronkhite-Canada syndrome, Nelson's syndrome, porphyria and haemochromatosis.
